# EUROPEAN PATENT SPECIFICATION

(11) **EP 4 468 975 B1**
(45) Date of publication and mention of the grant of the patent: **24.06.2026**
(21) Application number: 23707860.5
(22) Date of filing: 19.01.2023
(51) Int. Cl.: A61B 17/32, A61B 18/12, A61B 18/14, A61B 18/00, A61B 18/16

(54) **SURGICAL INSTRUMENT WITH MONOPOLAR AND ULTRASONIC FUNCTIONALITY**
CHIRURGISCHES INSTRUMENT MIT MONOPOLARER UND ULTRASCHALLFUNKTION
INSTRUMENT CHIRURGICAL DOTÉ D'UNE FONCTIONNALITÉ MONOPOLAIRE ET ULTRASONORE

(30) Priority: 29.01.2022 CN 202210112395
(43) Date of publication of application: 04.12.2024
(73) Proprietor: Reach Surgical, Inc., Tianjin 300462 (CN); West China Hospital of Sichuan University, Chengdu, Sichuan 610041 (CN)
(72) Inventor: WANG, Ziqiang, Chengdu, Sichuan 610047 (CN); ZHANG, Xiaoqiang, Tianjin 300462 (CN); HUANG, Jin, Chengdu, Sichuan 610047 (CN); YANG, Tinghan, Chengdu, Sichuan 610047 (CN); LI, Sandong, Tianjin 300462 (CN); FAN, Xiaoyun, Tianjin 300462 (CN)
(74) Representative: Bockhorni & Brüntjen Partnerschaft Patentanwälte mbB
(86) International application number: PCT/CN2023/073153
(87) International publication number: WO 2023/143373

(56) References cited:
- WO-A1-2022/195412
- US-A1- 2017 164 973
- US-A1- 2019 216 530
- US-A1- 2021 196 346
- US-A1- 2021 212 720

## Description

### TECHNICAL FIELD

The present disclosure relates to the field of surgical instruments, and more particularly, to a surgical instrument with monopolar and ultrasonic functionality.

### BACKGROUD OF THE INVENTION

Ultrasonic surgical instruments, such as ultrasonic scalpels, are used in many applications in surgical procedures. Depending upon specific device configurations and operational parameters, ultrasonic surgical instruments may provide substantially simultaneous transection and hemostasis by coagulation of tissue, desirably minimizing patient trauma.

Electrosurgical devices are configured to apply electrical energy on tissue in order to treat the tissue, and are also used in many surgical application scenes. An electrosurgical instrument comprises an end effector assembly mounted at a distal end, and the end effector assembly comprises one or more electrodes. The end effector assembly may be positioned against tissue such that electrical current is introduced into the tissue. The electrosurgical devices may be configured for bipolar or monopolar operation. During bipolar operation, current is introduced into and returned from the tissue by active and return electrodes of the end effector. During monopolar operation, current is introduced into the tissue by the active electrode of the end effector and returned through the return electrode (e.g., a grounding pad). The active electrode and the return electrode are separately located on a patient's body. Heat generated by the current flow through the tissue may form hemostatic seals within the tissue and/or between tissues and thus may be particularly useful for sealing blood vessels, for example. The end effector of the electrosurgical device sometimes also comprises a cutting member that is movable relative to the tissue and the electrodes to transect the tissue. Electrical energy applied by an electrosurgical device may be transmitted to the instrument by a generator. The electrical energy may be in the form of radio frequency ("RF") energy. RF energy is a form of electrical energy that may be in the frequency range of 300 kHz to 1 MHz.

At present, ultrasonic surgical instruments and monopolar electrosurgical instruments are two different kinds of surgical instruments, and there are also differences in usage scenarios and methods. When using an ultrasonic scalpel, an operator needs to operate a trigger of the ultrasonic surgical instrument to close a jaw, clamp a tissue to be transected/coagulated in the jaw, and then operate an excitation button to output ultrasonic energy, thus completing the operation on the tissue. In contrast, the monopolar electrosurgical instrument may transect/coagulate the tissue by only abutting an end effector mechanism thereof against the tissue to be operated. Therefore, the ultrasound surgical instruments are usually configured to complete the fine dissection of tissues and blood vessels, while the monopolar electrosurgical instruments may be configured to quickly transect omentum and fascia tissues. Furthermore, in the actual operation process, when ultrasonic surgical instruments are used, there will be bleeding and oozing of blood in tissues. However, ultrasonic surgical instruments need to close the jaws and clamp bleeding points before the tissues may be coagulated. In many cases, due to the poor clamping of tissues, surgeons usually use monopolar electrosurgical device for coagulation or sealing. In the process of changing instruments, the amount of bleeding in tissues will often increase, which makes it difficult for surgeons to find bleeding points and increases the risk of surgery. And, changing instruments many times will also affect the operation efficiency and prolong the operation time.

US 2019/216530 A1 discloses energy-based having end effector assemblies incorporating ultrasonic and electrosurgical functionality to facilitate treating, e.g., sealing and/or dissecting tissue.

Further surgical instruments are disclosed in US 2017/164973 A1,

US 2021/196346 A1, and US 2021/212720 A1. Post-published WO 2022/195412 A1 is prior art as per Article 54(3) EPC and discloses a surgical instrument according to the preamble of claim 1.

### SUMMARY OF THE INVENTION

The invention is defined by the subject matter of claim 1.

It is provided in the present disclosure that a surgical instrument comprises: a transmission assembly and an end effector assembly, wherein the transmission assembly is configured to transmit ultrasonic energy or electrosurgical energy provided by a generator to the end effector assembly, and the end effector assembly comprises: an ultrasonic blade configured to apply the ultrasonic energy to the tissue; and a clamp arm assembly operatively to be pivoted towards or away from the ultrasonic blade, wherein the clamp arm assembly comprises: a clamp pad arranged adjacent to the ultrasonic blade and configured to compress the tissue clamped therebetween; at least a portion of a first electrode arranged on a side of the clamp pad that is away from the ultrasonic blade and configured to apply the electrosurgical energy to the tissue; and an insulative support member pivotally coupled to the transmission assembly and configured to support the clamp pad and the first electrode.

The present disclosure further provides a surgical system, comprising a generator and the surgical instrument with monopolar and ultrasonic functionality, wherein the generator comprises an ultrasonic module and an electrosurgical module, and the surgical instrument is electrically connected to the ultrasonic module and the electrosurgical module through cables respectively.

### DESCRIPTION OF THE DRAWINGS

While the specification concludes with claims particularly pointing out and distinctly claiming the disclosure, it is believed that the disclosure will be better understood from the detailed description of certain embodiments thereof when read in conjunction with the accompanying drawings. Unless the context indicates otherwise, like numerals are used in the drawings to identify similar elements in the drawings. In addition, some of the figures may have been simplified by the omission of certain elements in order to more clearly show other elements. Such omissions are not necessarily indicative of the presence or absence of particular elements in any of the exemplary embodiments, except as may be explicitly stated in the corresponding detailed description.
FIG. 1 is a schematic structure diagram of a specific embodiment of a surgical instrument with monopolar and ultrasonic functionality according to the present disclosure;
FIG. 2 is an exploded view of a specific embodiment of the surgical instrument according to the present disclosure;
FIG. 3 is an exploded view of a specific embodiment of an end effector assembly and a distal end of a transmission assembly according to the present disclosure;
FIG. 4 is a schematic structural diagram of a specific embodiment of a first electrode in the end effector assembly according to the present disclosure;
FIG. 5 is a schematic structural diagram of another specific embodiment of the first electrode in the end effector assembly according to the present disclosure;
FIG. 6 is a schematic structural diagram of another specific embodiment of the first electrode in the end effector assembly according to the present disclosure;
FIG. 7 is a working state schematic diagram of another specific embodiment of the first electrode in the end effector assembly according to the present disclosure;
FIG. 8 is a storage state schematic diagram of another specific embodiment of the first electrode in the end effector assembly according to the present disclosure;
FIG. 9 is a connection relation schematic diagram of the end effector assembly according to the present disclosure;
FIG. 10 is a schematic structural diagram of a specific embodiment of a clamp arm according to the present disclosure;
FIG. 11 is a schematic structural diagram of a specific embodiment of an insulative support member according to the present disclosure;
FIG. 12 is a schematic structural diagram of a specific embodiment of the first electrode according to the present disclosure;
FIG. 13 is an exploded view of a specific embodiment of a handle assembly according to the present disclosure;
FIG. 14 is a sectional view of the specific embodiment of the handle assembly according to the present disclosure; and
FIG. 15 is a schematic structural diagram of a specific embodiment of a surgical system according to the present disclosure.

### DESCRIPTION OF EXEMPLARY EMBODIMENTS

The following detailed description describes examples of embodiments of the disclosure solely for the purpose of enabling one of ordinary skill in the relevant art to make and use the invention. As such, the detailed description and illustration of these embodiments are purely illustrative in nature and are in no way intended to limit the scope of the disclosure, or its protection, in any manner. It should also be understood that the drawings are not to scale and in certain instances details have been omitted, which are not necessary for an understanding of the present disclosure.

In the description of the present disclosure, it should be noted that, the orientation or positional relationships indicated by the terms "center", "up", "down", "left", "right", "vertical", "horizontal", "inside", "outside" and the like are orientation or positional relationships based on the accompanying drawings, which are only for convenience and simplification of the description of the present disclosure, but are not intended to indicate or imply that the indicated device or element must have a specific orientation, be constructed and operated in a specific orientation, and thus, may not be understood as a limitation to the present disclosure. Moreover, the terms "first", "second" and "third" are used for descriptive purposes only and may not be understood as indicating or implying relative importance.

In the description of the present disclosure, it should be noted that unless otherwise specified and limited, the terms "mounting", "connection", "connected", "coupled" and the like should be understood broadly, for example, the connection may be fixed connection, and may also be detachable connection or integral connection; may be direct connection, may also be indirect connection through an intermediate medium, and may also be internal communication of two elements. The specific meanings of the above terms in the present disclosure may be understood by those of ordinary skills in the art according to specific conditions.

In addition, the technical features involved in different embodiments of the present disclosure described below may be combined with each other as long as they do not constitute conflicts with each other.

In various embodiments of the present disclosure, "distal end/side" refers to an end/side of a surgical instrument that is far away from an operator when the surgical instrument is operated, and "proximal end/side" refers to an end/side that is close to the operator when the surgical instrument is operated.

The present disclosure provides a surgical instrument with monopolar and ultrasonic functionality (hereinafter referred to as surgical instrument). The surgical instrument shall be applied for open surgery, laparoscopic surgery or transection surgery, and the like. The surgical instrument is operatively to be electrically connected with a generator 100 via a wire. The generator 100 may be provided with an ultrasonic module 50 and an electrosurgical module 60, so as to output ultrasonic energy and/or electrosurgical radio frequency energy, such that ultrasonic energy and/or electrosurgical energy may be transmitted to an end effector assembly 40, and the end effector assembly 40 may be operated to perform ultrasonic vibration or monopolar radio frequency output. It should be noted that the surgical instrument may also be electrically connected to the ultrasonic generator and the electrosurgical generator via wires, respectively, so as to operatively transmit ultrasonic energy and/or electrosurgical energy to the end effector assembly 40. When the end effector assembly 40 outputs ultrasonic vibration, the surgical instrument may be used to clamp, coagulate and/or cut or transect tissues. The clamp arm assembly 42 may be operatively unfolded, so that the end effector assembly 40 may be used without clamping, in order to apply ultrasonic energy to tissues. When the end effector assembly 40 performs outputting of monopolar radio frequency energy, the end effector assembly 40 may be positioned against the tissue, so that current is introduced into the tissue to coagulate or cut the tissue.

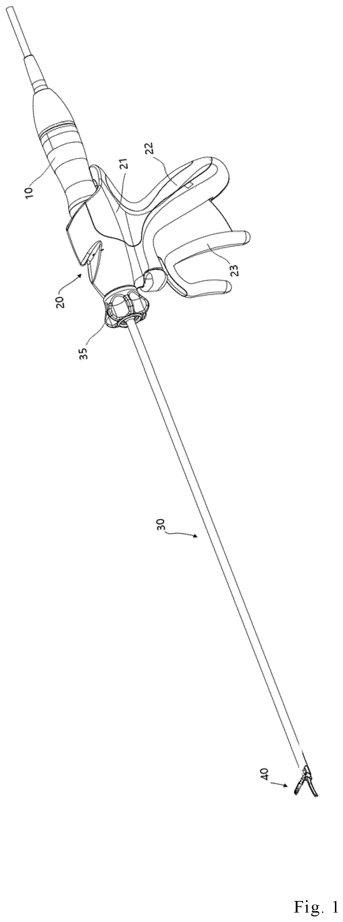

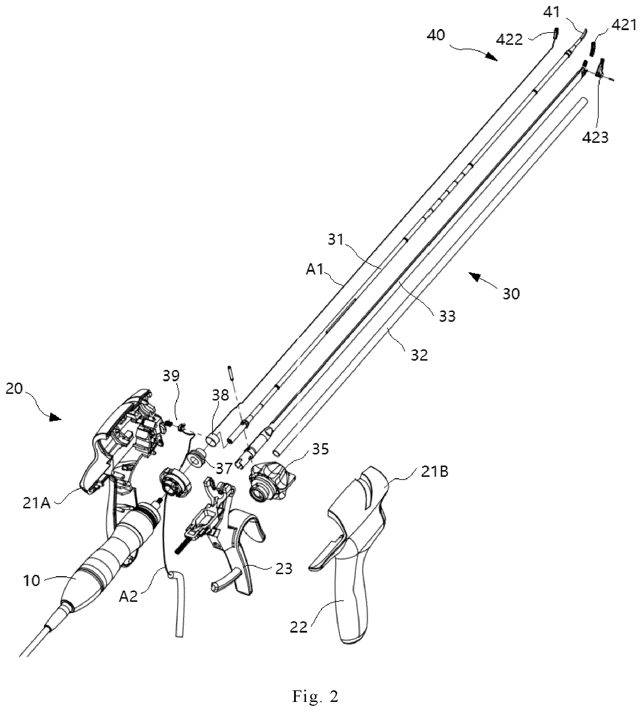

Referring to FIG. 1 and FIG. 2, the surgical instrument comprises an ultrasonic transducer 10, a handle assembly 20, a transmission assembly 30, and an end effector assembly 40. The ultrasonic transducer 10 is operatively connected to the ultrasonic module 50 through a wire (also known as a cable), which converts electrical energy from the ultrasonic module 50 into ultrasonic vibration that is further transmitted to the end effector assembly 40 through the transmission assembly 30. The handle assembly 20 is designed for an operator to operate the surgical instrument. For example, the handle assembly 20 is adapted to actuate the end effector assembly 40 through the transmission assembly 30, so as to cut and/or seal the tissue. The handle assembly 20 may be grasped by the operator in various manners. The end effector assembly 40 is operatively closed through a trigger of the surgical instrument. By inserting the ultrasonic transducer 10 into the handle assembly 20, a proximal portion of the surgical instrument receives and is assembled with a distal end of the ultrasonic transducer 10. By way of example, generator 50 and transducer 10 in the depicted embodiment are configured to generate a standing vibrational wave having a frequency of about 55 kHz. However, various other ultrasonic frequencies may be employed, such as between about 20 and about 120 kHz.

Details of the end effector assembly 40, the transmission assembly 30 and the handle assembly 20 of the surgical instrument are respectively described below.

### End effector assembly

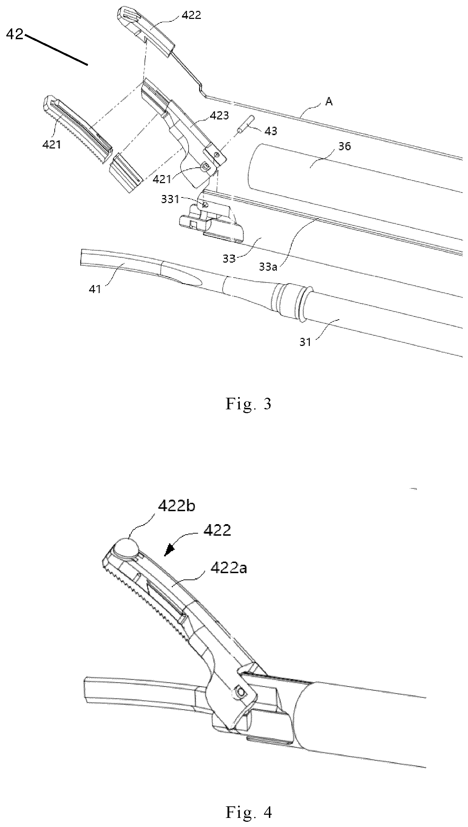

As shown in FIG. 3, the end effector assembly 40 of the surgical instrument comprises an ultrasonic blade 41 and a clamp arm assembly 42 that may be operatively actuated for pivotal movement towards or away from the ultrasonic blade 41, so as to close or open the end effector assembly 40. The clamp arm assembly 42 may be actuated to move between an open position, a clamping position and a cutting position. In the open position, for example, at least a part of the clamp arm assembly 42 is actuated to be spaced from the ultrasonic blade 41 and does not contact with the tissue. In the clamp position, at least a part of the clamp arm assembly 42 is actuated to be spaced apart from the ultrasonic blade 41 and urges against the tissue. In the cutting position, the clamp arm assembly 42 urges against the ultrasonic blade 41 to cut the tissue clamped therebetween.

As shown in FIG. 3, the clamp arm assembly 42 comprises a clamp pad 421, a first electrode 422 and an insulative support member 423 configured to support or receive the clamp pad 421 and the first electrode 422.

The clamp pad 421 is located on a side of the clamp arm assembly 42 that is adjacent to the ultrasonic blade 41, which is made of polymer material with a low friction coefficient, or may be formed of any other suitable low friction material. The clamp pad 421 is such arranged so as to cooperate with the ultrasonic blade 41 for clamping the tissue, and pivotal movement of the clamp arm assembly 42 causes the clamp pad 421 substantially to be parallel to and in contact with the ultrasonic blade 41, thereby defining a tissue treatment area. Therefore, the tissue is clamped between the clamp pad 421 and the ultrasonic blade 41. As shown in the figures, the clamp pad 421 may be provided with an uneven surface (e.g., a serrated structure) so as to enhance the clamping of the tissues clamped between the clamp pad 421 and the ultrasonic blade 41. The serrated structure or tooth may provide traction against the movement of the blade. The tooth may also provide opposite traction to the blade and the clamp movement. As may be understood by those skilled in the art, the serrated structure is only one example of various tissue engaging surfaces used to prevent the movement of the tissue relative to the ultrasonic blade 41. Other exemplary embodiments comprise protrusions, cross patterns, tread patterns, shot blast or sandblasted surfaces, etc.

At least a portion of the first electrode 422 is arranged on a side of the clamp pad 421 that is away from the ultrasonic blade 41, for example, at least a portion of the first electrode 422 is extending outwardly from the clamp pad 421, as shown in FIG. 3. The first electrode 422 is configured to be operatively electrically connected to a monopolar electrosurgical device 60 so as to apply electrosurgical energy to the tissues, wherein the first electrode 422 is made of metal materials, and the insulative support member 423 is made of non-conductive insulating materials (such as ceramics and PVC) and is pivotally coupled to the transmission assembly 30 to isolate the electrical conduction between the first electrode 422 and the transmission assembly 30.

When the surgical instrument is operated to cut tissue through ultrasonic energy, the clamp arm assembly 42 of the end effector assembly 40 is actuated to pivot towards the ultrasonic blade 41 to cut the tissue clamped therebetween. When the tissue is bleeding, the first electrode 422 on the clamp arm assembly 42 is adapted to coagulate or seal the bleeding point, thereby reducing the tissue bleeding as well as improving the operation efficiency. Or, in a scene suitable for treatment with monopolar electrosurgical instruments, the first electrode 422 is adapted to be applied on corresponding tissue, for example, to cut and/or coagulate the tissue more efficiently, so as to shorten the operation time and improve the operation efficiency.

When the first electrode 422 is adapted to coagulate the tissue (for example, a vessel), in an alternative embodiment, a distal end of the first electrode 422 may be directly applied on the vessel. In one of embodiments of the present disclosure, the first electrode 422 is designed to be tapered from bottom to top, or from proximal end to the distal end thereof, for the purpose of more precis cut and/or coagulation.

In order to coagulate the bleeding point of the tissue conveniently and control an acting area, alternatively, as shown in FIG. 4 to FIG. 8, the first electrode 422 comprises an electrode body 422a and a working member 422b located at a distal end thereof. The working member 422b is adapted to be operatively contact with the tissue, and the working member 422b is electrically connected to the electrode body 422a. The working member 422b may be designed in various manners, which shall not be limited to the implementations described in detail below, since for those skilled in the art, changes or variations may be made on the basis of the implementations provide in the present disclosure.

One implementation of the electrode body 422a and the working member 422b is shown in FIG. 4 to FIG. 6. The electrode body 422a is shaped as an approximately rectangular metal block, and the working member 422b is located on an end face of the electrode body 422a far away from the clamp pad 421. The working member 422b and the electrode body 422a may be detachably or fixedly connected by welding, riveting or fasteners, or the working member 422b is directly integrated with the electrode body 422a by casting, forging or machining. To treat a smaller tissue treatment surface more accurately, the working member 422b is shaped into a hemispherical shape, a conical shape, a truncated cone shape or a pyramid shape, so that a smaller end of the working member 422b acts on a tissue to be coagulated and a damaged area of the tissue may be controlled.

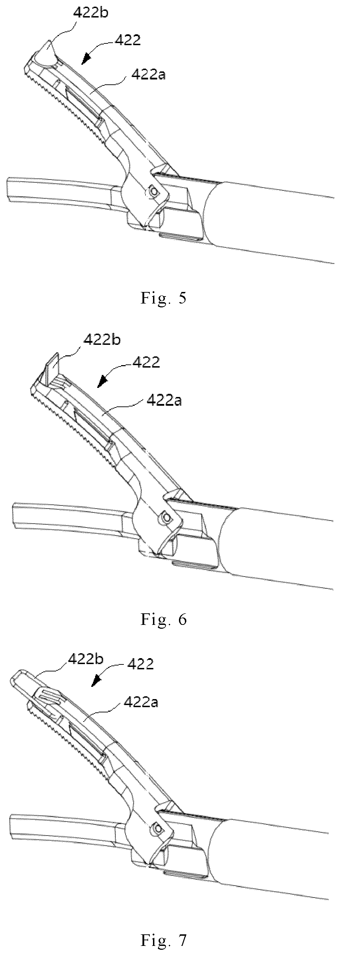

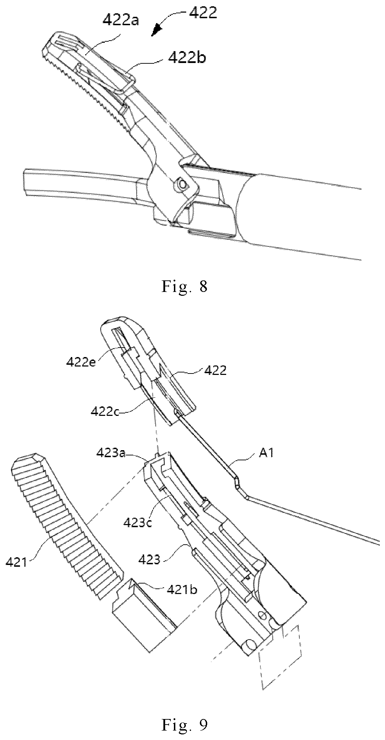

An alternative implementation of the electrode body 422a and the working member 422b is shown in FIG. 7 and FIG. 8. The working member 422b is pivotally connected to the electrode body 422a, and has a folded position and a unfolded position. When the working member 422b is in the unfolded position, as shown in FIG. 7, the working member 422b extends out of a distal end of the electrode body 422a. When the working member 422b is in the folded position, as shown in FIG. 8, the working member 422b is located inside of the distal end of the electrode body 422a. More particularly, left and right sides of the electrode body 422a have pivot holes, the working member 422b is shaped as a ferrule structure formed by bending a metal wire, and an end of an opening side of the ferrule structure is pivotally connected to the pivot holes of the electrode body 422a. When the surgical instrument performs ultrasonic transecting, the ferrule structure pivots toward a proximal end of the electrode body 422a and overlaps an upper side of the electrode body 422a. When the surgical instrument uses the first electrode 422 to coagulate or transect tissue, the ferrule structure pivots toward a distal end of the electrode body 422a and extends out of the electrode body 422a, forming a ring structure with the distal end of the electrode body 422a, and a smaller tissue may be sleeved in the ring structure for transecting or coagulating.

In another alternative implementation of the electrode body 422a and the working member 422b, the working member 422b is telescopically connected to the electrode body 422a, and has a folded position and a unfolded position. In the unfolded position, the working member 422b extends out a distal end of the electrode body 422a. In the folded position, the working member 422b is located inside of the distal end of the electrode body 422a. More particularly, the electrode body 422a is provided with an cavity, and the working member 422b is telescopically connected into the cavity through an elastic member. When the surgical instrument coagulates or transects tissue by the first electrode 422, the working member 422b pops out of the cavity and acts on the tissue. In this way, the working member 422b is shaped into a hemispherical, conical, truncated cone shaped or pyramid shaped block structure, so that the smaller end of the working member 422b acts on a tissue to be coagulated and a damaged area of the tissue may be controlled.

In the clamp arm assembly 42, the connection mode among the first electrode 422, the insulative support member 423 and the clamp pad 421 is not limited to be unique. In an alternative embodiment, a proximal end of the first electrode 422 is connected to the insulative support member 423, and a distal end of the first electrode 422 is connected to the clamp pad 421. By respectively connecting the proximal and distal ends of the first electrode 422 with the clamp pad 421 and the insulative support member 423, the structural stability of the first electrode 422 is improved.

The connection mode among the first electrode 422, the insulative support member 423 and the clamp pad 421 is not limited to be unique. In one mode, the first electrode 422 and the clamp pad 421 may be welded and then integrally connected to the insulative support member 423 by adhesive bonding, or by fusion welding, brazing, solid phase diffusion, or the like, or by self-propagating high-temperature synthesis, instantaneous liquid phase, and the like.

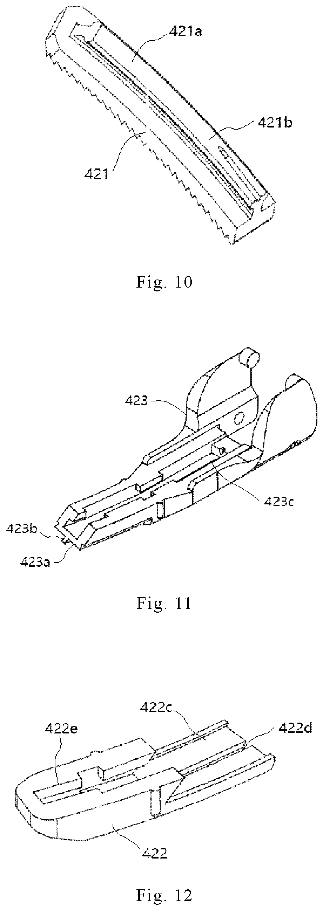

In another alternative embodiment, as shown in FIG. 9, the first electrode 422 and the insulative support member 423 are docked by a first plug-in structure, and the first electrode 422 and the clamp pad 421 are spliced by a second plug-in structure. As shown in FIG. 11 and FIG. 12, an inner surface of the proximal end of the first electrode 422 is provided with a dovetail groove 422c, and a distal end of the insulative support member 423 is provided with a dovetail bulge 423a matched with the dovetail groove 422c. A first docking groove 422d extending along a longitudinal axis of the surgical instrument is arranged on a groove surface of the dovetail groove 422c at the proximal end of the first electrode 422, and a first docking bulge 423b matched with the first docking groove 422d is arranged at the distal end of the insulative support member 423. The docking of the first electrode 422 and the insulative support member 423 may be realized by the mating of the dovetail groove 422c and the dovetail bulge 423a and the mating of the first docking groove 422d and the first docking bulge 423b. An inner surface of the distal end of the first electrode 422 is provided with a second docking groove 422e, and a surface of the clamp pad 421 facing the first electrode 422 is provided with a second docking bulge 421a, the second docking bulge 421a is inserted into the second docking groove 422e to realize the docking between the first electrode 422 and the clamp pad 421.

The clamp pad 421 and the insulative support member 423 are docked by a third plug-in structure. Specifically, as shown in FIG. 10 to FIG. 12, a surface of the clamp pad 421 facing the insulative support member 423 is provided with a third docking bulge 421b, an inner surface of the insulative support member 423 is provided with a third docking groove 423c, and the third docking bulge 421b is docked into the third docking groove 423c. Both of the second docking bulge 421a and the third docking bulge 421b on the clamp pad 421 are integrally-formed structures and extend along the longitudinal axis of the surgical instrument, and cross sections of the second docking bulge 421a and the third docking bulge 421b are the same in size and shape.

When the first electrode 422, the clamp pad 421 and the insulative support member 423 are assembled, the insulative support member 423 slides along an upper surface of the clamp pad 421 from a proximal end to a distal end until the proximal end of the clamp pad 421 abuts against a blocking surface of the insulative support member 423. Then, the first electrode 422 slides from the distal end to the proximal end of the clamp pad 421, so that the proximal end of the first electrode 422 is docked with the insulative support member 423, and the distal end of the first electrode 422 is docked with the clamp pad 421, realizing the fixed connection of the three, and then pivotally connecting the clamp arm assembly 42 to the distal end of the transmission assembly 30.

### Transmission assembly

As shown in FIG. 2, it is provided in the present disclosure an endoscopic instrument and the transmission assembly 30 may be configured as an elongated shaft extending distally away from the handle assembly 20 of the instrument. The transmission assembly 30 comprises a waveguide 31 for transmitting the ultrasonic energy provided by the ultrasonic transducer 10 to the ultrasonic blade 41, an inner tube 32 sleeved on the waveguide 31, and an outer tube 33 sleeved on the inner tube 32. The outer tube 33 is axially mounted relative to the handle assembly 20, and the clamp arm assembly 42 is pivotally coupled to the outer tube 33. The proximal portion of the inner tube 32 is coupled with an actuation mechanism of the handle assembly 20, and the distal portion is couple to the clamp arm assembly 42. The inner tube 32 is actuated through the actuation mechanism so as to be reciprocated axially relative to the outer tube 33, and further actuate the clamp arm assembly 42 to pivot about the outer tube 33.

In various embodiments, the waveguide 31 may be made from a variety of materials, particularly various medically and surgically acceptable metals such as titanium, titanium alloy (for example, Ti6Al4V), aluminum, aluminum alloy or stainless steel. In some embodiments, such as the embodiments shown in the drawings, the ultrasonic blade 41 and the waveguide 31 are formed as a single unit, for example, such as fabricated from a single metal rod that has been milled so as to provide the desired features. Alternately, the waveguide 31 and the ultrasonic blade 41 may comprise two or more separable components of the same of differing compositions, with the components coupled to one another by, for example, adhesive, welding, a threaded stud, and/or other suitable ways known to those skilled in the art. For example, the ultrasonic blade 41 may be connected to the waveguide (42) by a threaded connection, a welded joint, or other coupling mechanisms.

The waveguide 31 is configured so that, during use, the distal end of the ultrasonic blade 41 will be disposed at (or near) a vibrational anti-node of the system in order to tune the acoustic assembly to a desired resonant frequency when the end effector is not under load (i.e., not in contact with tissue). When the transducer 10 is energized, the distal end of the ultrasonic blade 41 will vibrate longitudinally, for example, in a peak-to-peak range of about 10 µm to 100 µm, for example, at a predetermined vibration frequency f₀ of 55,500 Hz, and optionally transversely. **In** various embodiments, the ultrasonic blade 41 vibrates longitudinally in a range of about 20 µm to about 90 µm.

As shown in FIG. 3, the pivotal movement of the clamp arm assembly 42 relative to the ultrasonic blade 41 is realized by setting a pair of pivot points on the clamp arm assembly 42. The pair of pivot points are respectively coupled with the outer tube 33 and the inner tube 32. The outer tube 33 is fixedly coupled to the handle assembly 20. The clamp arm assembly 42 is pivotally coupled to the outer tube 33 via a first through hole 424 on the clamp arm assembly 42 and a corresponding second through hole 331 on the outer tube 33. A fastening pin 43 or rivet slides through the first through hole 424 and the second through hole 331 to pivotally connect the clamp arm assembly 42 to the outer tube 33. The inner tube 32 moves along a longitudinal axis of the outer tube 33. A pivot pin on the clamp arm assembly 42 engages a pivot hole 321 at a distal end of the inner tube 32. Therefore, the reciprocating movement of the inner tube 32 relative to the outer tube 33 makes the clamp arm assembly 42 pivot relative to the ultrasonic blade 41.

As shown in FIG. 3, the transmission assembly 30 further comprises a sheath 36 sleeved on an outer wall of the outer tube 33. A wire of the first electrode 422 passes between the sheath 36 and the outer tube 33, so that a proximal end of the wire of the first electrode 422 is located inside the handle assembly 20, and the wire extends into the surgical instrument from the handle assembly 20 side, and extends along the transmission assembly 30 to the end effector assembly 40 side.

In an alternative embodiment, the sheath 36 may be an elastic rubber sleeve, and the wire is located in the elastic rubber sleeve to keep the position of the wire, so as to avoid a problem that the wire is broken due to movement. Alternatively, an groove 33a extending along the longitudinal axis is formed on the outer wall of the outer tube 33, and the wire of the first electrode 422 is located in the groove 33a, which further limits the position of the wire and prevents the wire from shifting relative to the outer tube 33 and the sheath 36, so that the wire may rotate with the outer tube 33.

### Handle assembly

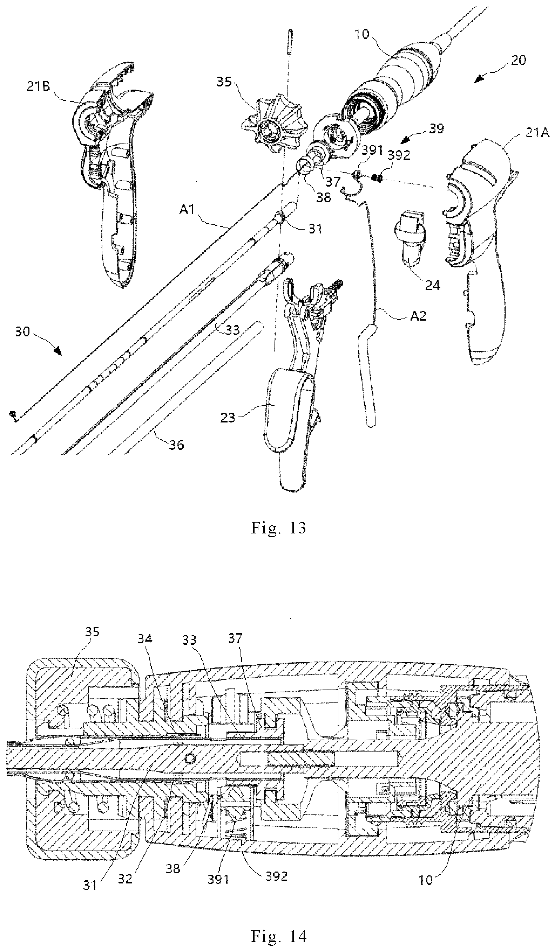

As shown in FIG. 13, the handle assembly 20 comprises a main housing 21 and a handgrip 22 downwardly extending from the main housing 21. The handle assembly 20, especially the handgrip 22 thereof, is adapted for being held by a medical practitioner, so that during use in order to facilitate grasping and manipulation of the instrument, while isolating the operator from the ultrasonic vibrations. A trigger 23 is supported on the handle assembly 20 for pivotal movement towards and away from the handgrip 22 to cause pivotal movement of the clamp arm assembly 42 located at the distal end of the transmission assembly 30. Specifically, the movement of the trigger 23 towards the handgrip 22 actuates the inner tube 32 to move proximally, thereby pivoting the clamp arm assembly 42 towards the ultrasonic blade 41. A triggering action provided by the trigger 23 and the cooperative handgrip 22 helps to conveniently and effectively manipulate and position the instrument, and operates the clamp arm assembly 42 at the distal end of the instrument to pivot toward the ultrasonic blade 41 side, whereby the tissue is effectively driven and pushed onto the ultrasonic blade 41. The movement of the trigger 23 away from the handgrip 22 actuates the inner tube 32 to move distally, thereby pivoting the clamp arm assembly 42 away from the ultrasonic blade 41. The handle assembly 20 is further provided with a hand switch 24 used to control operation of the instrument, for example, allowing the ultrasonic generator to output, so as to cause the ultrasonic blade 41 to vibrate. The tissue is clamped by the end effector assembly 40 (i.e., applies pressure to the tissue) during closing of the end effector assembly 40, and at the same time, transects the tissue/stops the bleeding of the tissue under the ultrasonic energy action. As an alternative embodiment of handle shown in the drawings, the handle assembly 20 may be provided with various forms, such as the scissor arrangement of the handgrip 22. A proximal portion of the main housing 21 is open so as to receive the ultrasonic transducer 10 to be inserted therein.

The handle assembly 20 comprises a first half housing 21A and a second half housing 21B which are matched with each other. The first half housing 21A and the second half housing 21B are connected together along a seam in a variety of ways known in the prior art, such as welding, gluing, snap connection, and the like. It may be understood that the handle assembly 20 may be an integrated structure, or a structure in which more than two matched parts are connected together in various ways. The handle assembly 20 and the trigger 23 may be made of suitable plastic materials which may be sterilized or other sterilized materials such as various metal materials.

Proximal ends of the waveguide 31, the outer tube 33 and the inner tube 32 are connected to each other through a bayonet connector assembly located in the main housing 21, so that the waveguide 31, the outer tube 33 and the inner tube 32, as a whole, may rotate together with the ultrasonic transducer 10 relative to the handle assembly 20by means of a knob 35. The waveguide 31 extends into the main housing 21 of the handle assembly 20 through the knob 35. The bayonet connector assembly comprises a slip ring 37 located at a proximal end of the bayonet connector assembly, wherein the slip ring 37 is sleeved on the proximal end of the outer tube 33 and rotatably connected to the main housing 21. The bayonet connector assembly further comprises a support ring 34 located at a distal end of the bayonet connector assembly, wherein the support ring 34 is used to support the inner tube 32 and connect the inner tube with the manipulating mechanism, and the manipulating mechanism triggers the inner tube 32 to reciprocate along a longitudinal axis thereof to drive the clamp arm assembly 42 to open or close. The waveguide 31 is fixedly connected to the ultrasonic transducer 10 through connecting rods provided with threads on both sides. In use, the outer tube 33 and the waveguide 31 may be rotated by the knob 35, so that the end effector assembly 40 and the clamp arm assembly 42 connected thereto are adjusted to a required direction. In use, the rotation of the knob 35 relative to the handle assembly 20 causes the outer tube 33, the waveguide 31 and the ultrasonic transducer 10 operatively connected thereto to rotate relative to the handle assembly 20. In the described embodiment, the knob 35 is also used to keep a part of the connector assembly in the handle assembly 20, and thus, keep the surgical instrument in an assembled state.

The wire (A) of the cable between the first electrode 422 and the electrosurgical module 60 extends from the handgrip 22 side, and extends along the main housing 21 and the transmission assembly 30 to the end effector assembly 40. Rotation of the knob 35 relative to the handle assembly 20 causes the outer tube 33 to rotate relative to the handle assembly 20 correspondingly, the wire (A) arranged on the transmission assembly 30 rotates accordingly. In order that the electric signal transmission between the first electrode 422 and the electrosurgical module 60 is not affected by the rotation of the transmission assembly 30, the wire (A) comprises a first section of wire (Al) and a section second section of wire (A2), wherein the first section of wire (Al) is arranged extending along a longitudinal axis direction of the transmission assembly 30, and a distal end of the first section of wire is connected to the first electrode 422. The second section of wire (A2) is arranged extending along an extension direction of the handgrip 22, that is, the second section of wire (A2) and the first section of wire (A1) form an included angle of about 90 degrees, and the first section of wire (A1) and the second section of wire (A2) are connected by an electrical connection assembly 39. By using the electrical connection assembly 39, the two sections of wires (A) are electrically connected, so that the first section of wire (A1) may still be electrically connected to the second section of wire (A2) when rotating with the transmission assembly 30, while the second section of wire (A2) is fixed relative to the position of the handgrip 22.

The bayonet connector assembly comprises a conductive ring 38 sleeved on the slip ring 37, and a proximal end of the first section of wire (A1) is located between the conductive ring 38 and the slip ring 37. The electrical connection assembly 39 abuts on the conductive ring 38, so as to keep in electrical contact with the conductive ring 38, thereby realizing the electrical connection between the first section of wire (A1) and the second section of wire (A2).

As shown in FIG. 13 and FIG. 14, the electrical connection assembly 39 comprises a conductive terminal 391 and an elastic member 392, the conductive terminal 391 is connected to the second section of wire A2, the elastic member 392 is located between the main housing 21 and the conductive terminal 391, and an elastic force of the elastic member 392 makes the conductive terminal 391 abut against the conductive ring 38. Specifically, an inner wall of the main housing 21 is provided with a mounting base 21a, the mounting base 21a is shaped as a sliding sleeve structure having an open cavity, and the conductive terminal 391 is slidably connected to the mounting base 21a. A compression spring is arranged in the mounting base 21a, one end of the compression spring is sleeved on the conductive terminal 391, and the other end of the compression spring abuts against the inner wall of the main housing 21. An elastic force of the compression spring acts on the conductive terminal 391, so that the other end of the conductive terminal always abuts on the conductive ring 38, thus realizing the electrical connection between the conductive terminal and the conductive ring. One side of the conductive terminal 391 facing the conductive ring 38 is shaped as an arc-shaped groove matched with an outer wall of the conductive ring 38, so that the contact between the two is more stable and the electrical connection reliability is higher.

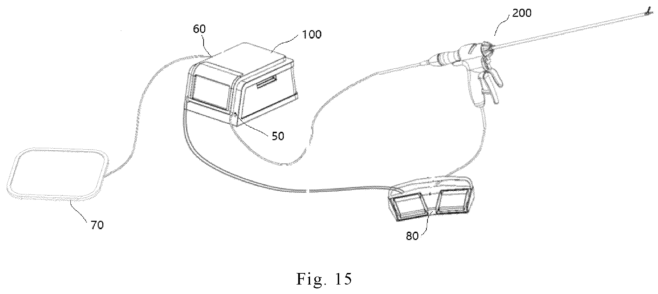

As shown in FIG. 15, a specific embodiment of a surgical system of the present disclosure comprises a generator 100. The generator 100 integrates an ultrasonic module 50 and an electrosurgical module 60, as well as the surgical instrument 200 with the electrosurgical monopolar and ultrasonic functionality of one of embodiments of the present disclosure. The surgical instrument 200 is operatively to be connected to the ultrasonic module 50 and the electrosurgical module 60 through cable(s) respectively.

The surgical system further comprises a second electrode pad 70 (also known as return pad), a polarity of the second electrode pad 70 is opposite to that of the first electrode 422, and the second electrode pad 70 is connected to the electrosurgical module 60 through a wire A. During operation, the second electrode pad 70 is positioned under the patient, so that a tissue to be operated is located between the first electrode 422 and the second electrode pad 70 to form a conductive loop. When the operator uses the surgical instrument to output electrosurgical energy, the first electrode 422 of the clamp arm assembly 42 is applied on the tissue to for coagulation or transection of the tissue, which is convenient for operators to operate.

In an alternative embodiment, the generator 100 is provided with a first switch, and the first switch is configured to enable the ultrasonic module 50 and/or the electrosurgical module 60. For example, the first switch is provided with two modes, i.e., an enable mode and a disable mode, to enable or disable functionality of the ultrasonic module 50 and the electrosurgical module 60. It should be understood that more modes could be provided in alternative embodiment, so as to enable or disable the ultrasonic module 50 and the electrosurgical module 60 respectively.

To facilitate the control of the electrosurgical energy output of the first electrode 422, the surgical system further comprises a second switch. The second switch is adapted to turn on or off the electrical connection between the electrosurgical module 60 and the first electrode 422. In this way, the operator may independently control the output of the first electrode 422 through the second switch.

In an alternative embodiment, the second switch is configured as a footswitch 80, and the electrical connection between the electrosurgical module 60 and the first electrode 422 will be turned on/off through stepping on the footswitch 80. Therefore, both footswitch operation and hand switch operation are provided to the operator, so that the operator may not mis-operate the surgical instrument of the present disclosure. Meanwhile, the footswitch 80 may be actuated by stepping on with foot, which facilitate the operator to operates the surgical instrument, such that the operator may operate the surgical instrument by means of hand switch and footswitch, together or respectively.

In an alternative embodiment of the present disclosure, the ultrasonic generator and the electrosurgical generator are provided respectively, which are electrically connected to the surgical instrument through the cable(s), such that the end effector assembly may operatively energized by ultrasonic vibration or monopolar electrosurgical output. During use, the clamp arm assembly of the end effector assembly is actuated to pivot towards the ultrasonic blade so as to transect the tissue clamped therebetween; when the tissue is bleeding, the first electrode on the clamp arm assembly may be used to coagulate the bleeding point, no needs for changing another suitable surgical instrument (e.g. an monopolar instrument), thereby reducing the tissue bleeding and improving the operation efficiency. Furthermore, the surgical instrument provided in anyone of embodiments of the present disclosure shall be adapted for monopolar coagulation or transection of the tissue, individually, which is convenient for surgeons to operate.

## Claims

1. A surgical instrument (200) comprising:
a transmission assembly (30) and an end effector assembly (40), wherein the transmission assembly (30) is configured to transmit ultrasonic energy or electrosurgical energy provided by a generator (100) to the end effector assembly (40), and the end effector assembly (40) comprises:
an ultrasonic blade (41) configured to apply the ultrasonic energy to the tissue;
and a clamp arm assembly (42) operatively pivoted towards or away from the ultrasonic blade (41), wherein the clamp arm assembly (42) comprises:
a clamp pad (421) arranged adjacent to the ultrasonic blade (41) and configured to compress the tissue clamped therebetween;
a first electrode (422) disposed on a side of the clamp pad (421) away from the ultrasonic blade (41) and configured to apply the electrosurgical energy to the tissue;
and
an insulative support member (423) pivotally coupled to the transmission assembly (30) and configured to support the clamp pad (421) and the first electrode (422);
wherein the first electrode (422) comprises an electrode body (422a) and a working member (422b) supported at a distal portion thereof, wherein the working member (422b) is operatively to be applied to the tissue, and the working member (422b) is electrically connected to the electrode body (422a);
wherein the working member (422b) is arranged on the electrode body (422a) opposing from the clamp pad (421);
**characterised in that** the working member (422b) is adapted to be actuated between a folded position and an unfolded position; wherein the working member (422b) extends out of a distal end of the electrode body (422a) when being located in the unfolded position; and the working member (422b) is received in the distal portion of the electrode body (422a) when being located in the folded position;
and that the working member (422b) is pivotally connected to the electrode body (422a).

2. The surgical instrument (200) according to claim 1, wherein the working member (422b) is shaped as a ferrule structure having an opening, and an opening side of the ferrule structure is pivotally connected to the electrode body (422a).

3. The surgical instrument (200) according to claim 1, wherein a proximal portion of the first electrode (422) is supported on the insulative support member (423), and a distal portion thereof is coupled with the clamp pad (421).

4. The surgical instrument (200) according to claim 3, wherein the first electrode (422) and the insulative support member (423), and/or the first electrode (422) and the clamp pad (421), and/or the clamp pad (421) and the insulative support member (423) are docked.

5. The surgical instrument (200) according to claim 1, wherein the transmission assembly (30) comprises:
a waveguide (31) configured to transmit the ultrasonic energy, wherein the ultrasonic blade (41) extends distally from a distal end thereof;
an inner tube (32) sleeved on the waveguide (31), and a distal end of the inner tube (32) is coupled with the clamp arm assembly (42);
an outer tube (33) sleeved on the inner tube (32), and the clamp arm assembly (42) is pivotally coupled to a distal end of the outer tube (33); and
the inner tube (32) is operatively to be actuated with respect to the outer tube (33) so as to actuate the clamp arm assembly (42) pivoting with respect to the ultrasonic blade (41).

6. The surgical instrument (200) according to claim 5, wherein the transmission assembly (30) further comprises a sheath (36) that is sleeved on the outer tube (33), and a partial wire for the first electrode (422) is located between the sheath (36) and the outer tube (33).

7. The surgical instrument (200) according to claim 6, wherein an outer wall of the outer tube (33) is provided with a groove (33a) extending longitudinally, and a wire for the first electrode (422) is located in the groove (33a).

8. The surgical instrument (200) according to claim 6, further comprising a handle assembly (20) that comprises: a main housing (21), wherein a proximal end of the main housing (21) is provided with an ultrasonic transducer (10) connected to a generator (100), a distal end of the main housing (21) is provided with a knob (35), and the transmission assembly (30) is rotatably connected to the main housing (21) through the knob (35);
a handgrip (22) extending downward from the main housing (21) and configured to be held by a user; and
a trigger (23) configured to operatively actuate the clamp arm assembly (42) pivoting towards or away from the ultrasonic blade (41).

9. The surgical instrument (200) according to claim 8, wherein a wire (A) between the first electrode (422) and the generator (100) extends from the handle side, and extends along the main housing (21) and the transmission assembly (30) to the end effector assembly (40).

10. The surgical instrument (200) according to claim 9, wherein the wire (A) comprises a first section of wire (A1) and a second section of wire (A2), wherein the first section of wire (A1) is arranged extending along a longitudinal axis direction of the transmission assembly (30); and the second section of wire (A2) is arranged extending along an extension direction of the handle, and the first section of wire (A1) and the second section of wire (A2) are connected by an electrical connection assembly (39).

11. The surgical instrument (200) according to claim 10, wherein proximal ends of the outer tube (33) and the inner tube (32) are connected by a bayonet connector assembly, and the bayonet connector assembly comprises:
a slip ring (37), wherein the slip ring (37) is sleeved on the proximal end of the outer tube (33) and rotatably connected to the main housing (21); and
a conductive ring (38) sleeved on the slip ring (37), wherein a proximal end of the first section of wire (A1) is located between the conductive ring (38) and the slip ring (37).

## Patentansprüche

1. Chirurgisches Instrument (200), umfassend:
eine Übertragungsbaugruppe (30) und eine Endeffektorbaugruppe (40), wobei die Übertragungsbaugruppe (30) so konfiguriert ist, dass sie von einem Generator (100) bereitgestellte Ultraschallenergie oder elektrochirurgische Energie an die Endeffektorbaugruppe (40) überträgt, und die Endeffektorbaugruppe (40) umfasst:
eine Ultraschallklinge (41), die dazu ausgelegt ist, die Ultraschallenergie auf das Gewebe aufzubringen;
und eine Klemmarmbaugruppe (42), die funktional in Richtung der Ultraschallklinge (41) oder von dieser weg schwenkbar ist, wobei die Klemmarmbaugruppe (42) umfasst:
ein Klemmkissen (421), das benachbart zur Ultraschallklinge (41) angeordnet und so konfiguriert ist, dass es das dazwischen geklemmte Gewebe komprimiert;
eine erste Elektrode (422), die auf einer von der Ultraschallklinge (41) abgewandten Seite des Klemmkissens (421) angeordnet und so konfiguriert ist, dass sie die elektrochirurgische Energie auf das Gewebe aufbringt; und
ein isolierendes Stützelement (423), das schwenkbar mit der Übertragungsbaugruppe (30) verbunden ist und dazu ausgelegt ist, das Klemmkissen (421) und die erste Elektrode (422) zu stützen;
wobei die erste Elektrode (422) einen Elektrodenkörper (422a) und ein an einem distalen Abschnitt davon abgestütztes Arbeitselement (422b) umfasst, wobei das Arbeitselement (422b) funktional auf das Gewebe aufbringbar ist und das Arbeitselement (422b) elektrisch mit dem Elektrodenkörper (422a) verbunden ist;
wobei das Arbeitselement (422b) auf dem Elektrodenkörper (422a) gegenüber dem Klemmkissen (421) angeordnet ist;
**dadurch gekennzeichnet, dass** das Arbeitselement (422b) so ausgelegt ist, dass es zwischen einer gefalteten Position und einer entfalteten Position betätigt werden kann; wobei sich das Arbeitselement (422b) aus einem distalen Ende des Elektrodenkörpers (422a) heraus erstreckt, wenn es sich in der entfalteten Position befindet; und das Arbeitselement (422b) in dem distalen Abschnitt des Elektrodenkörpers (422a) aufgenommen ist, wenn es sich in der zusammengeklappten Position befindet;
und dass das Arbeitselement (422b) schwenkbar mit dem Elektrodenkörper (422a) verbunden ist.

2. Chirurgisches Instrument (200) nach Anspruch 1, wobei das Arbeitselement (422b) als eine eine Öffnung aufweisende Hülsenstruktur ausgebildet ist und eine Öffnungsseite der Hülsenstruktur schwenkbar mit dem Elektrodenkörper (422a) verbunden ist.

3. Chirurgisches Instrument (200) nach Anspruch 1, wobei ein proximaler Abschnitt der ersten Elektrode (422) auf dem isolierenden Stützelement (423) abgestützt ist und ein distaler Abschnitt davon mit dem Klemmkissen (421) verbunden ist.

4. Chirurgisches Instrument (200) nach Anspruch 3, wobei die erste Elektrode (422) und das isolierende Stützelement (423) und/oder die erste Elektrode (422) und das Klemmkissen (421) und/oder das Klemmkissen (421) und das isolierende Stützelement (423) miteinander verbunden sind.

5. Chirurgisches Instrument (200) nach Anspruch 1, wobei die Übertragungsbaugruppe (30) umfasst:
einen Wellenleiter (31), der zur Übertragung der Ultraschallenergie ausgebildet ist, wobei sich die Ultraschallklinge (41) von dessen distalen Ende distal erstreckt;
ein Innenrohr (32), das auf den Wellenleiter (31) aufgeschoben ist, wobei ein distales Ende des Innenrohrs (32) mit der Klemmarmbaugruppe (42) verbunden ist;
ein Außenrohr (33), das auf das Innenrohr (32) aufgeschoben ist, wobei die Klemmarmbaugruppe (42) schwenkbar mit einem distalen Ende des Außenrohrs (33) verbunden ist; und
das Innenrohr (32) in Bezug auf das Außenrohr (33) funktional betätigbar ist, um die Klemmarmbaugruppe (42) in Bezug auf die Ultraschallklinge (41) schwenkbar zu betätigen.

6. Chirurgisches Instrument (200) nach Anspruch 5, wobei die Übertragungsbaugruppe (30) ferner eine Hülle (36) umfasst, die auf das Außenrohr (33) aufgeschoben ist, und ein Teil eines Drahtes für die erste Elektrode (422) zwischen der Hülle (36) und dem Außenrohr (33) angeordnet ist.

7. Chirurgisches Instrument (200) nach Anspruch 6, wobei eine Außenwand des Außenrohrs (33) mit einer sich in Längsrichtung erstreckenden Nut (33a) versehen ist und ein Draht für die erste Elektrode (422) in der Nut (33a) angeordnet ist.

8. Chirurgisches Instrument (200) nach Anspruch 6, das ferner eine Griffbaugruppe (20) umfasst, die Folgendes umfasst: ein Hauptgehäuse (21), wobei ein proximales Ende des Hauptgehäuses (21) mit einem Ultraschallwandler (10) versehen ist, der mit einem Generator (100) verbunden ist, ein distales Ende des Hauptgehäuses (21) mit einem Drehknopf (35) versehen ist, und die Übertragungsbaugruppe (30) über den Knopf (35) drehbar mit dem Hauptgehäuse (21) verbunden ist;
einen Handgriff (22), der sich vom Hauptgehäuse (21) nach unten erstreckt und so ausgebildet ist, dass er von einem Benutzer gehalten werden kann; und
einen Auslöser (23), der so ausgebildet ist, dass er die Klemmarmbaugruppe (42) betätigt, die sich in Richtung der Ultraschallklinge (41) hin oder von dieser weg schwenkt.

9. Chirurgisches Instrument (200) nach Anspruch 8, wobei sich ein Draht (A) zwischen der ersten Elektrode (422) und dem Generator (100) von der Griffseite aus erstreckt und entlang des Hauptgehäuses (21) und der Übertragungsbaugruppe (30) bis zur Endeffektorbaugruppe (40) verläuft.

10. Chirurgisches Instrument (200) nach Anspruch 9, wobei der Draht (A) einen ersten Drahtabschnitt (A1) und einen zweiten Drahtabschnitt (A2) umfasst, wobei der erste Drahtabschnitt (A1) so angeordnet ist, dass er sich entlang einer Längsachsenrichtung der Übertragungsbaugruppe (30) erstreckt; und der zweite Drahtabschnitt (A2) so angeordnet ist, dass er sich entlang einer Ausdehnungsrichtung des Griffs erstreckt, und der erste Drahtabschnitt (A1) und der zweite Drahtabschnitt (A2) durch eine elektrische Verbindungsbaugruppe (39) verbunden sind.

11. Chirurgisches Instrument (200) nach Anspruch 10, wobei die proximalen Enden des Außenrohrs (33) und des Innenrohrs (32) durch eine Bajonettverbindungsbaugruppe verbunden sind und die Bajonettverbindungsbaugruppe umfasst:
einen Gleitring (37), wobei der Gleitring (37) auf das proximale Ende des Außenrohrs (33) aufgeschoben und drehbar mit dem Hauptgehäuse (21) verbunden ist; und
einen auf den Gleitring (37) aufgeschobenen leitfähigen Ring (38), wobei sich ein proximales Ende des ersten Drahtabschnitts (A1) zwischen dem leitfähigen Ring (38) und dem Gleitring (37) befindet.

## Revendications

1. Instrument chirurgical (200) comprenant :
un ensemble de transmission (30) et un ensemble effecteur terminal (40), dans lequel l'ensemble de transmission (30) est configuré pour transmettre une énergie ultrasonique ou une énergie électrochirurgicale fournie par un générateur (100) à l'ensemble effecteur terminal (40), et l'ensemble effecteur terminal (40) comprend :
une lame à ultrasons (41) configurée pour appliquer l'énergie ultrasonique sur le tissu ; et
un ensemble bras de serrage (42) pivoté de manière fonctionnelle en direction ou en éloignement de la lame à ultrasons (41), dans lequel l'ensemble bras de serrage (42) comprend :
un tampon de serrage (421) agencé de manière adjacente à la lame à ultrasons (41) et configuré pour comprimer le tissu serré entre ceux-ci ;
une première électrode (422) disposée sur un côté du tampon de serrage (421) en éloignement de la lame à ultrasons (41) et configurée pour appliquer l'énergie électrochirurgicale sur le tissu ; et
un élément de support isolant (423) couplé de manière pivotante à l'ensemble de transmission (30) et configuré pour supporter le tampon de serrage (421) et la première électrode (422) ;
dans lequel la première électrode (422) comprend un corps d'électrode (422a) et un élément de travail (422b) supporté à une portion distale de celui-ci, l'élément de travail (422b) étant appliqué de manière fonctionnelle sur le tissu, et l'élément de travail (422b) étant connecté électriquement au corps d'électrode (422a) ;
dans lequel l'élément de travail (422b) est agencé sur le corps d'électrode (422a) à l'opposé du patin de serrage (421) ;
**caractérisé en ce que**
l'élément de travail (422b) est adapté pour être actionné entre une position pliée et une position dépliée ; dans lequel l'élément de travail (422b) s'étend à l'extérieur d'une extrémité distale du corps d'électrode (422a) quand il est situé dans la position dépliée ; et l'élément de travail (422b) est reçu dans la portion distale du corps d'électrode (422a) quand il est situé dans la position pliée ;
et **en ce que** l'élément de travail (422b) est connecté de manière pivotante au corps d'électrode (422a).

2. Instrument chirurgical (200) selon la revendication 1, dans lequel l'élément de travail (422b) est formé sous forme de structure à virole ayant une ouverture, et un côté d'ouverture de la structure à virole est connecté de manière pivotante au corps d'électrode (422a).

3. Instrument chirurgical (200) selon la revendication 1, dans lequel une portion proximale de la première électrode (422) est supportée sur l'élément de support isolant (423), et une portion distale de celle-ci est couplée au patin de serrage (421).

4. Instrument chirurgical (200) selon la revendication 3, dans lequel la première électrode (422) et l'élément de support isolant (423), et/ou la première électrode (422) et le patin de serrage (421), et/ou le patin de serrage (421) et élément du support isolant (423) sont assemblés.

5. Instrument chirurgical (200) selon la revendication 1, dans lequel l'ensemble de transmission (30) comprend :
un guide d'onde (31) configuré pour transmettre l'énergie ultrasonique, dans lequel la lame à ultrasons (41) s'étend de manière distale par rapport à une extrémité distale de celui-ci ;
un tube intérieur (32) emmanché sur le guide d'onde (31), et une extrémité distale du tube intérieur (32) étant couplée à l'ensemble bras de serrage (42) ;
un tube extérieur (33) emmanché sur le tube intérieur (32), et l'ensemble bras de serrage (42) étant couplé de manière pivotante à une extrémité distale du tube extérieur (33) ; et
le tube intérieur (32) doit être actionné de manière fonctionnelle par rapport au tube extérieur (33) de manière à actionner l'ensemble bras de serrage (42) en pivotement par rapport à la lame à ultrasons (41).

6. Instrument chirurgical (200) selon la revendication 5, dans lequel l'ensemble de transmission (30) comprend en outre une gaine (36) qui est emmanchée sur le tube extérieur (33), et un fil partiel pour la première électrode (422) est situé entre la gaine (36) et le tube extérieur (33).

7. Instrument chirurgical (200) selon la revendication 6, dans lequel une paroi extérieure du tube extérieur (33) est munie d'une rainure (33a) s'étendant longitudinalement, et un fil pour la première électrode (422) est situé dans la rainure (33a).

8. Instrument chirurgical (200) selon la revendication 6, comprenant en outre un ensemble poignée (20) comprend : un boîtier principal (21), dans lequel une extrémité proximale du boîtier principal (21) est munie d'un transducteur à ultrasons (10) connecté à un générateur (100), une extrémité distale du boîtier principal (21) est munie d'un bouton (35), et l'ensemble de transmission (30) est connecté en rotation au boîtier principal (21) par l'intermédiaire du bouton (35) ;
une partie de préhension (22) s'étendant vers le bas depuis le boîtier principal (21) et configurée pour être tenue par un utilisateur ; et
un déclencheur (23) configuré pour actionner de manière fonctionnelle l'ensemble bras de serrage (42) en pivotement en direction ou en éloignement de la lame à ultrasons (41).

9. Instrument chirurgical (200) selon la revendication 8, dans lequel un fil (A) entre la première électrode (422) et le générateur (100) s'étend depuis le côté poignée, et s'étend le long du boîtier principal (21) et de l'ensemble de transmission (30) jusqu'à l'ensemble effecteur terminal (40).

10. Instrument chirurgical (200) selon la revendication 9, dans lequel le fil (A) comprend une première section de fil (A1) et une deuxième section de fil (A2), la première section de fil (A1) étant agencée dans le prolongement d'une direction axiale longitudinale de l'ensemble de transmission (30) ; et la deuxième section de fil (A2) est agencée dans le prolongement d'une direction d'extension de la poignée, et la première section de fil (A1) et la deuxième section de fil (A2) sont connectées par un ensemble de connexion électrique (39).

11. Instrument chirurgical (200) selon la revendication 10, dans lequel des extrémités proximales du tube extérieur (33) et du tube intérieur (32) sont connectées par un ensemble connecteur à baïonnette, et l'ensemble connecteur à baïonnette comprend :
un contact tournant (37), le contact tournant (37) étant emmanché sur l'extrémité proximale du tube extérieur (33) et connecté en rotation au boîtier principal (21) ; et
une bague conductrice (38) emmanchée sur le contact tournant (37), une extrémité proximale de la première section de fil (A1) étant située entre la bague conductrice (38) et le contact tournant (37).
